# EUROPÄISCHE PATENTANMELDUNG

(11) **EP 1 834 942 A1**
(43) Veröffentlichungstag der Anmeldung: **19.09.2007**
(21) Anmeldenummer: 07003879.9
(22) Anmeldetag: 26.02.2007
(51) Int. Cl.: C07C 41/18, C07C 43/11, C08G 65/321

(54) **Verfahren zur Herstellung von längerkettigen Polyalkylenglykoldiethern**

(30) Priorität: 09.03.2006 DE 102006010940
(71) Anmelder: CLARIANT INTERNATIONAL LTD., 4132 Muttenz (CH)
(72) Erfinder: Stankowiak, Achim, Dr., 84503 Altötting (DE); Snell, Alexander, Dr., 4055 Basel (CH)
(74) Vertreter: Mikulecky, Klaus

(57) **Zusammenfassung**

Gegenstand der Erfindung ist ein Verfahren zur Herstellung von Alkylenglykoldiethern der Formel (I) 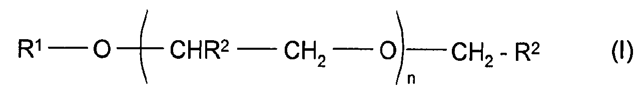 durch Umsetzung von Verbindungen der Formel (II) 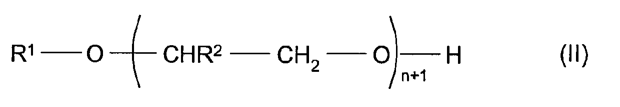 worin R¹ Wasserstoff oder C₁- bis C₃-Alkyl, R² Wasserstoff, CH₃ oder CH₂-CH₃ und n eine Zahl von 5 bis 500 bedeuten, in flüssiger Phase bei Temperaturen zwischen 170 und 300°C, in Gegenwart eines Raney-Nickel-Katalysators, der, bezogen auf das Gesamtgewicht des Katalysators, 0,1 bis 10 Gew.-% eines oder mehrerer anderer Metalle, ausgewählt aus den Nebengruppen I, VI und VIII des Periodensystems der Elemente, enthält.

## Beschreibung

Die vorliegende Erfindung betrifft ein Verfahren zur Herstellung von kettenförmigen Alkylenglykoldiethern mit einem Molekulargewicht von mindestens 250 g/mol.

Alkylenglykoldiether werden seit langer Zeit als polare, aprotische, inerte Lösungsmittel eingesetzt. Hochmolekulare Alkylenglykoldiether finden vor allem Anwendung in der Elektrochemie, als hochsiedende Lösungsmittel und als lineare Kronenether in der Phasen-Transfer-Katalyse.

Zu ihrer Herstellung werden so genannte indirekte Verfahren wie beispielsweise die Williamson-Ether-Synthese (K. Weissermel, H. J. Arpe "Industrielle Organische Chemie", 1998, Seite 179) oder die Hydrierung von Diglykoletherformal (DE-OS 24 34 057) industriell angewandt oder beschrieben. Beide Verfahren haben allerdings Nachteile: die zweistufige Williamson-Ether-Synthese ist durch den stöchiometrischen Chlor- und Alkaliverbrauch, sowie die Abtrennung des Reaktionswassers und entstehenden Natriumchlorids wenig ökonomisch. Die Hydrierung von Formal wird unter Hochdruck durchgeführt, was hohe Investitionen in den Anlagenbau voraussetzt und daher nicht für geringere Produktionsmengen geeignet ist.

Bei den sogenannten direkten Verfahren wird Alkylenoxid in einen kettenförmigen Ether in Gegenwart von Lewis-Säuren wie BF₃ (US-PS 4 146 736 und DE-OS 26 40 505 in Verbindung mit DE-OS 31 28 962) oder SnCl₄ (DE-OS 30 25 434) insertiert. Der Nachteil dieser Verfahren besteht darin, dass unvermeidlich größere Mengen an cyclischen Nebenprodukten, wie beispielsweise Dioxan oder Dioxolan, gebildet werden. Ferner lassen sich diese Verfahren nicht auf längerkettige Polyalkylenglykolether übertragen (hoher Anteil von Nebenprodukten).

Eine alternative Synthesemöglichkeit ist die katalytische Deformylierung von Glykolen und Methylglykolen:

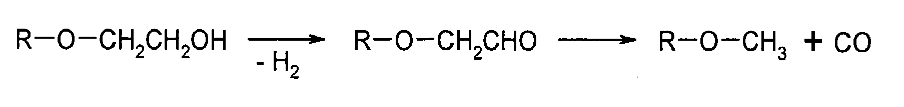

Die Patentschrift DE 2 900 279 beschreibt erstmals diesen Syntheseweg durch die Umsetzung von Polyethylenglykolen oder Polyethylenglykolmonomethylethern in der Gasphase bei 250-500°C in Gegenwart von geträgerten Palladium-, Platin-, Rhodium, Ruthenium- oder Iridium-Katalysatoren und Wasserstoff. Die japanische Patentschrift JP 60028429 beschreibt die Umsetzung von C₄- und längerkettigeren Monoalkylethern unter Verwendung eines Nickel/Rhenium-Katalysators geträgert auf γ-Aluminiumoxid. Auch bei diesem Verfahren wird kontinuierlich Wasserstoff zugeführt. Ebenfalls bekannt ist die Hydrierung von sekundären Hydroxylgruppen mit Wasserstoff bei Normaldruck unter Verwendung von Nickel-Trägerkatalysatoren (DE-OS 38 02 783). Bei diesem Verfahren gelingt die Synthese ausdrücklich nicht, wenn Raney-Nickel verwendet wird.

Aus der Patentschrift US 3 428 692 ist bekannt, dass durch Erhitzen von C₆- bis C₁₂-kettigen Monoalkyl- und Monophenylethern auf 200-300°C in Gegenwart von Nickel- und Cobalt-Katalysatoren die entsprechenden deformylierten methylblockierten Ethoxylate hergestellt werden können. Dabei entstehen jedoch Gemische von den gewünschten Methylethern mit nicht vollständig umgesetzten Ethoxylaten und 20-30 % nicht identifizierten Aldehydverbindungen. EP 0 043 420 beschreibt ein ähnliches Verfahren unter Verwendung von Palladium-, Platin- oder Rhodium-Katalysatoren, geträgert auf Al₂O₃ oder SiO₂.

Alle im derzeitigen Stand der Technik beschriebenen Verfahren sind entweder wenig selektiv oder aber technisch sehr aufwendig und daher unwirtschaftlich für die Herstellung von längerkettigen Alkylenglykoldiethern. Die sich hieraus ergebende Aufgabe wurde erfindungsgemäß entsprechend den Angaben des Anspruchs gelöst.

Überraschenderweise können längerkettige Alkylenglykole und Alkylenglykolmonoether in einem einfachen Slurry-Prozess durch Metall-Katalyse zu den gewünschten Alkylenglykoldiethern umgesetzt werden. Die Synthese gelingt quantitativ (> 99 %) und ohne Bildung von Nebenprodukten. Nach der Umsetzung kann der Katalysator in einem einfachen Filtrationsschritt vollständig abgetrennt werden (< 1 ppm Metall).

Gegenstand der Erfindung ist somit ein Verfahren zur Herstellung von Alkylenglykoldiethern der Formel (I) 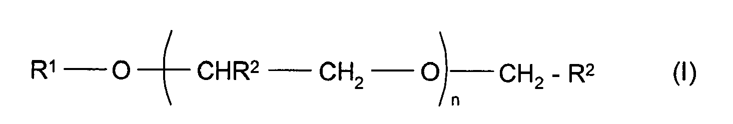 durch Umsetzung von Verbindungen der Formel (II) 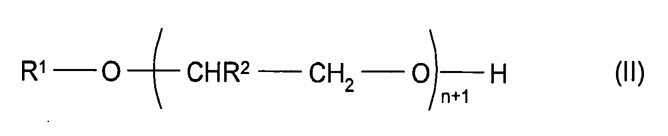 worin R¹ Wasserstoff oder C₁- bis C₃-Alkyl, R² Wasserstoff, CH₃ oder CH₂-CH₃ und n eine Zahl von 5 bis 500 bedeuten, in flüssiger Phase bei Temperaturen zwischen 170 und 300°C, in Gegenwart eines Raney-Nickel-Katalysators, der, bezogen auf den als elementares Metall berechneten Gesamtmetallgehalt des Katalysators, 0,1 bis 50 Gew.-% eines oder mehrerer anderer Metalle, ausgewählt aus den Nebengruppen I, VI und VIII des Periodensystems der Elemente, enthält.

Die Umsetzung an den Katalysatoren erfolgt vorzugsweise bei 200 bis 250°C. Die Reaktion führt man im Allgemeinen bei Normaldruck durch, man kann jedoch auch im Unter- oder Überdruck arbeiten. Die Reaktionszeit liegt im Allgemeinen zwischen 4 und 10 Stunden.
R¹ bedeutet vorzugsweise H oder Methyl.
R² bedeutet vorzugsweise Wasserstoff.
n ist vorzugsweise eine Zahl von 15 bis 300.

Der Raney-Nickel-Katalysator enthält vorzugsweise 0,2 bis 25, insbesondere 0,5 bis 10 Gew.-% eines oder mehrerer anderer Metalle ausgewählt aus den Nebengruppen I, VI und VIII des Periodensystems der Elemente. Bevorzugte Metalle aus den Nebengruppen I, VI und VIII des Periodensystems der Elemente sind Palladium, Eisen, Molybdän, Kupfer, Chrom, Cobalt, Platin, Rhodium, Ruthenium und Iridium. Diese Metalle können entweder mit dem Raney-Nickel auf demselben Trägermaterial dotiert, oder auf einem separaten Trägermaterial dem Katalysator beigefügt sein. Im Fall der Mischung von Katalysatoren auf separaten Trägern ist mit dem Begriff Katalysator die Mischung gemeint. Der Metallgehalt des Katalysators ist stets in Prozenten des Gesamtmetallgehalts angegeben. Das als elementares Metall berechnete Gesamtmetallgewicht des Katalysators entspricht 100 Gew.-%.

Das erfindungsgemäße Verfahren wird nun an einigen Beispielen noch näher erläutert:

### Beispiel 1:

### Synthese von Polyglykoldimethylether mit einem Molgewicht von ca. 500 g/mol

In einem 250 ml Dreihalskolben werden unter Schutzgas 361,7g Polyglykol monomethylether (Molgewicht ca. 500 g/mol), 12,3 g Palladium auf Aktivkohle (Palladiumgehalt 0,6 g) und 19,4 g wasserfreier Raney-Nickel (Nickelgehalt 9,7 g) bei 230°C stark gerührt. Nach 8 Stunden Reaktionszeit wird das Reaktionsgemisch bei 80°C über Kieselgel filtriert. Der Umsatz beträgt 98,6 %. Im Produkt kann kein Nickel (AAS) oder Palladium (ICPOES) mehr nachgewiesen werden.

### Beispiel 2:

### Synthese von Polyglykoldimethylether mit einem Molgewicht von ca. 2000 g/mol

In einem 250 ml Dreihalskolben werden unter Schutzgas 399,5 g Polyglykol monomethylether (Molgewicht ca. 2000 g/mol) 19,7 g Palladium auf Aktivkohle (Palladiumgehalt 0,98 g) und 31,0 g wasserfreier Raney-Nickel (Nickelgehalt 15,5 g) bei 230°C stark gerührt. Nach 6 Stunden Reaktionszeit wird das Reaktionsgemisch bei 80°C über Kieselgel filtriert. Der Umsatz beträgt 99,3 %. Im Produkt kann kein Nickel oder Palladium mehr nachgewiesen werden.

### Beispiel 3:

### Synthese von Polyglykoldimethylether mit einem Molgewicht von ca. 4000 g/mol

In einem 250 ml Dreihalskolben werden unter Schutzgas 395,5 g Polyglykol monomethylether (Molgewicht ca. 4000 g/mol) 19,4 g Palladium auf Aktivkohle (Palladiumgehalt 0,97 g) und 30,6 g wasserfreier Raney-Nickel (Nickelgehalt 15,3 g) bei 230°C stark gerührt. Nach 8 Stunden Reaktionszeit wird das Reaktionsgemisch bei 80°C über Kieselgel filtriert. Der Umsatz beträgt 98,8 %.

### Beispiel 4 (Vergleich):

### Synthese von Polyglykoldimethylether mit einem Molgewicht von ca. 10000 g/mol

In einem 250 ml Dreihalskolben werden unter Schutzgas 331,5 g Polyglykolmonomethylether (Molgewicht ca. 10000 g/mol) und 18,7 g wasserfreier Raney-Nickel (Nickelgehalt 9,4 g) bei 200°C stark gerührt. Nach 8 Stunden Reaktionszeit wird das Reaktionsgemisch bei 80°C über Kieselgel filtriert. Der Umsatz beträgt 86,1 %.

### Beispiel 5:

### Synthese von Polyglykoldimethylether mit einem Molgewicht von ca. 10000 g/mol

In einem 250 ml Dreihalskolben werden unter Schutzgas 332,4 g Polyglykol monomethylether (Molgewicht ca. 10000 g/mol) 11,6 g Palladium auf Aktivkohle (Palladiumgehalt 0,58 g) und 18,3 g wasserfreier Raney-Nickel (Nickelgehalt 9,2 g) bei 230°C stark gerührt. Nach 8 Stunden Reaktionszeit wird das Reaktionsgemisch über Kieselgel filtriert. Der Umsatz beträgt 99,0 %.

### Beispiel 6:

### Synthese von Polyglykoldimethylether mit einem Molgewicht von ca. 10000 g/mol

Analog zu Beispiel 5 werden 332,4 g Polyglykol monomethylether (Molgewicht ca. 10000 g/mol) 18,3 g Raney-Kupfer (Kupfergehalt 9,2 g) und 18,3 g Raney-Nickel (Nickelgehalt 9,2 g) bei 200°C stark gerührt. Nach 8 Stunden Reaktionszeit wird das Reaktionsgemisch über Kieselgel filtriert. Der Umsatz beträgt 89,2 %.

### Beispiel 7:

### Synthese von Polyglykoldimethylether mit einem Molgewicht von ca. 10000 g/mol

Analog zu Beispiel 5 werden 332,0 g Polyglykol monomethylether (Molgewicht ca. 10000 g/mol) 18,2 g Raney-Nickel (Nickelgehalt 9,1 g) dotiert mit 3 Gew.-% Chrom und 3 Gew.-% Eisen (bezogen auf das Gesamtgewicht an Metallen im Katalysator) bei 220°C stark gerührt. Nach 8 Stunden Reaktionszeit wird das Reaktionsgemisch über Kieselgel filtriert. Der Umsatz beträgt 89,2 %.

### Beispiel 8:

### Synthese von Polyglykoldimethylether mit einem Molgewicht von ca. 10000 g/mol

Analog zu Beispiel 5 werden 330,1 g Polyglykol monomethylether (Molgewicht ca. 10000 g/mol) 18,0 g Raney-Nickel (Nickelgehalt 9,0 g) dotiert mit 8 Gew.-% Kupfer und 3 Gew.-% Molybdän (bezogen auf das Gesamtgewicht an Metallen im Katalysator) bei 220°C stark gerührt. Nach 8 Stunden Reaktionszeit wird das Reaktionsgemisch über Kieselgel filtriert. Der Umsatz beträgt 93,4 %.

## Patentansprüche

1. Verfahren zur Herstellung von Alkylenglykoldiethern der Formel (I) 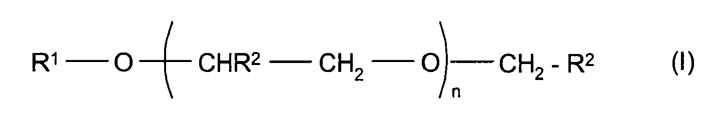 durch Umsetzung von Verbindungen der Formel (II) 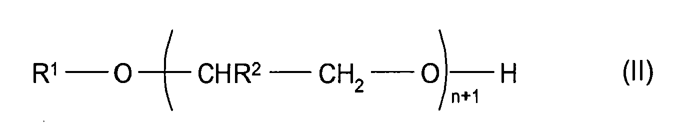 worin R¹ Wasserstoff oder C₁- bis C₃-Alkyl, R² Wasserstoff, CH₃ oder CH₂-CH₃ und n eine Zahl von 5 bis 500 bedeuten, in flüssiger Phase bei Temperaturen zwischen 170 und 300°C, in Gegenwart eines Raney-Nickel-Katalysators, der, bezogen auf das Gesamtgewicht des Katalysators, 0,1 bis 50 Gew.-% eines oder mehrerer anderer Metalle, ausgewählt aus den Nebengruppen I, VI und VIII des Periodensystems der Elemente, enthält.

2. Verfahren gemäß Anspruch 1, worin R¹ H oder Methyl bedeutet.

3. Verfahren gemäß Anspruch 1 und/oder 2, worin R² Wasserstoff bedeutet.

4. Verfahren gemäß einem oder mehreren der Ansprüche 1 bis 3, worin n eine Zahl von 15 bis 300 bedeutet.

5. Verfahren gemäß einem oder mehreren der Ansprüche 1 bis 4, worin der Raney-Nickel-Katalysator 0,2 bis 25 Gew.-% eines oder mehrerer anderer Metalle ausgewählt aus den Nebengruppen I, VI und VIII des Periodensystems der Elemente enthält.

6. Verfahren gemäß einem oder mehreren der Ansprüche 1 bis 5, worin die Metalle aus den Nebengruppen I, VI und VIII des Periodensystems der Elemente aus Palladium, Eisen, Molybdän, Kupfer, Chrom, Cobalt, Platin, Rhodium, Ruthenium und Iridium ausgewählt sind.

7. Verfahren gemäß einem oder mehreren der Ansprüche 1 bis 6, worin die Metalle aus den Nebengruppen I, VI und VIII des Periodensystems der Elemente mit dem Raney-Nickel auf dem selben Trägermaterial dotiert, oder auf einem separaten Trägermaterial dem Katalysator beigefügt sind.
